# EUROPÄISCHE PATENTSCHRIFT

(11) **EP 1 089 720 B1**
(45) Veröffentlichungstag und Bekanntmachung des Hinweises auf die Patenterteilung: **02.05.2002**
(21) Anmeldenummer: 99927905.2
(22) Anmeldetag: 08.06.1999
(51) Int. Cl.: A61K 9/70

(54) **TRANSDERMALES THERAPEUTISCHES SYSTEM, ENTHALTEND HORMONE UND KRISTALLISATIONSINHIBITOREN**
TRANSDERMAL THERAPEUTIC SYSTEM CONTAINING HORMONES AND CRYSTALLIZATION INHIBITORS
SYSTEME THERAPEUTIQUE TRANSDERMIQUE, CONTENANT DES HORMONES ET DES INHIBITEURS DE CRISTALLISATION

(30) Priorität: 25.06.1998 DE 19828274
(43) Veröffentlichungstag der Anmeldung: 11.04.2001
(73) Patentinhaber: Rottapharm B.V., 1017 PS Amsterdam (NL)
(72) Erfinder: ROVATI, Lucio, I-20052 Monza (IT); SANTORO, Antonino, I-20052 Monza (IT)
(74) Vertreter: Flaccus, Rolf-Dieter, Dr.
(86) Internationale Anmeldenummer: EP9903923
(87) Internationale Veröffentlichungsnummer: WO9966907

(56) Entgegenhaltungen:
- EP-A- 0 848 950
- EP-A- 0 848 960
- EP-A- 0 913 158

## Beschreibung

Die Erfindung betrifft ein transdermales therapeutisches System (TTS) zur kontrollierten Abgabe von Estradiol in Kombination mit Norethisteronacetat an die menschliche Haut.

Estradiol in Kombination mit Norethisteronacetat zeigt in den zur Formulierung von transdermalen therapeutischen Systemen üblicherweise verwendeten Hilfsstoffen wie Polyacrylathaftklebern, Tackifiern, Weichmachern und Absorptionsverbesserern eine sehr geringe Sättigungslöslichkeit. Dadurch ist die Beladbarkeit von TTS mit gelöstem Wirkstoff stark begrenzt bzw. es treten während der Lagerung bei Übersättigung unerwünschte Kristallisationen auf. Dadurch wird der Anteil an gelösten Wirkstoffen in der Matrix reduziert, was sich negativ auf deren Freisetzung auswirkt.

Bei Kombinationspräparaten aus Estradiol und Norethisteronacetat wurden Anwendungsformen entwickelt, bei denen die Wirkstoffe in einem transdermalen therapeutischen System in räumlich getrennten Bereichen enthalten sind. Die Fertigung derartiger TTS ist jedoch sehr kostenaufwendig.

Das gemeinsame Einbringen von transdermalen therapeutischen Systemen mit Trocken-mitteln in die Primärpackung verringert die Gefahr der Rekristallisation, ist jedoch sehr aufwendig.

In DE- OS 43 36 557 ist ein wirkstoffhaltiges transdermales therapeutisches System auf Basis eines Haftklebers beschrieben, der Ester des Kolophoniums enthält. Die Herstellung erfolgt derart, daß die Komponenten bei Temperaturen zwischen 100 und 140 °C in der Schmelze geknetet und anschließend beschichtet werden. Diese hohen Temperaturen bei der Herstellung von Arzneiformen bergen die Gefahr, daß Abbauprodukte in einem unakzeptablen hohen Anteil gebildet werden können.

Die WO 95/30409 beschreibt ein topisches Polymer-Freisetzungssystem für die Verabreichung bestimmter Wirkstoffe mittels einer treibgasfreien Aerosolpumpe. Als Vorteil wird die Abwesenheit von Klebern herausgestellt. Als zusätzliche Komponenten werden Kristallisationsinhibitoren/Stabilisatoren und/oder Penetrationsenhancer wie substituierte Cyclodextrine, Transcutol, Harnstoff und Isoterpene verwendet, wobei die Wirkstoffkombination von Estradiol und Norethisteronacetat nicht beansprucht wird.

Aufgabe der Erfindung ist daher die Bereitstellung eines stabilen, d. h. rekristallisationsfreien Pflasters mit den Wirkstoffen Estradiol und Norethisteronacetat.

Überraschenderweise hat sich gezeigt, daß die Aufgabe durch ein transdermales therapeutisches System mit den Merkmalen des Hauptanspruches gelöst wird. Erfindungsgemäß wird als Kristallisationsinhibitor ein Terpolymer auf Basis von Butylmethacrylat, 2-Dimethylaminoethylmethacrylat und Methylmethacrylat, insbesondere im molaren Verhältnis von 1 : 2 : 1 verwendet.

Es hat sich gezeigt, daß sich als Kristallisationsinhibitor Eudragit E 100® (Firma Röhm), vorzugsweise in einem Anteil von 0,05 bis 30 Gew.-%, besonders eignet.

Durch Wasserstoffbrückenbildung zwischen den basischen Gruppen des Kristallisations-inhibitors und den beweglichen Wasserstoffatomen des Estradiol-Moleküls kommt es zu einer Immobilisierung von Estradiol. Dadurch wird die Konzentration an frei beweglichem Estradiol in der Matrix reduziert und die Kristallisation verhindert.

Das haftklebende Reservoir enthält Estradiol und Norethisteronacetat in einem Gewichtsverhältnis von 1 : 2 bis 1 : 15, vorzugsweise 1 : 3 bis 1 : 7 und einer Gesamtkonzentration von bis zu 25 Gew.-%.

Das Reservoir kann einen Bestandteil aus der Gruppe der Alterungsschutzmittel, Weichmacher, Antioxidantien und Absorptionsverbesserer, enthalten, wobei der Weichmacher in einer Konzentration von 0 bis 5 Gew.-% und das Alterungsschutzmittel in einer Konzentration von 0,1 bis 2 Gew.-% eingesetzt werden.

Geeignete Alterungsschutzmittel, Weichmacher, Antioxidantien und Absorptionsverbesserer sind dem Fachmann bekannt und beispielsweise in der DE 37 43 949 beschrieben.

Um das transdermale therapeutische System auf der Haut applizieren zu können, ist es erforderlich, daß dieses haftklebende Eigenschaften aufweist. Um diese Eigenschaften bei dem erfindungsgemäßen transdermalen therapeutischen System einzustellen, verwendet man Polyacrylathaftkleber in Form von Lösungen in organischen Lösungsmitteln, sogenannte Lösemittel-Haftkleber.

Weiterhin sind Polyacrylat-Haftkleber in Form von wäßrigen Dispersionen verwendbar.

Darüberhinaus sind auch Schmelzhaftkleber geeignet. Diese sind lösemittel- oder dispersionsmittelfrei und werden aus der Schmelze appliziert.

Weiterhin sind auch UV-vernetzbare Acrylathaftkleber geeignet. Diese sind lösemittelfrei und werden mit den üblichen Beschichtungsverfahren appliziert. Anschließend erfolgt durch Bestrahlung mit UV-Licht eine Vernetzung der Polymerketten. Dies ist erforderlich, um dem Haftkleber eine ausreichende Kohäsion zu geben.

Das Reservoir des transdermalen therapeutischen Systems kann aus mehreren Schichten bestehen,die gleiche oder unterschiedliche Wirkstoffkonzentrationen aufweisen können.

Die Schichtdicke des Reservoirs beträgt 0,02 mm bis 0,500 mm, vorzugsweise jedoch 0,030 mm bis 0,200 mm.

Das Reservoir kann mit einer zusätzlichen haftklebenden Schicht bzw. einem haftklebenden Rand versehen sein. Das wird dann erforderlich, wenn das Reservoir selbst nur unzureichende haftklebende Eigenschaften aufweist.

Das transdermale therapeutische System gemäß vorliegender Erfindung ist für therapeutische Zwecke in der Humanmedizin bestimmt.

Im folgenden wird die Erfindung anhand von Beispielen weiter erläutert.

### Beispiel 1

| | |
|---|---|
| 163,50 g | Durotak 387-2287 (Firma National Starch) (Polyacrylathaftkleber), |
| 0,48 g | Eudragit E 100 (Firma Röhm) (Polyacrylat) |

werden unter Rühren homogenisiert und mit der Aufschlämmung aus

| | |
|---|---|
| 2,17 g | Eutanol G (Firma Caesar und Loretz) (langkettiger Fettalkohol), |
| 0,03 g | Aluminiumacetylacetonat (Firma Merck-Schuchardt), |
| 1,25 g | Estradiol-Hemihydrat, |
| 8,33 g | Norethisteronacetat |

in dem Lösemittelgemisch aus

| | |
|---|---|
| 25,93 g | Ethylacetat und |
| 25,93 g | Ethanol |

versetzt. Es wird 24 Stunden bei Raumtemperatur gerührt.

Die so erhaltene Kleberlösung wird auf eine wiederablösbare Schutzschicht Hostaphan
RN 100, beidseitig silikonisiert, beschichtet, so daß nach dem Trocknen eine wirkstoffhaltige Matrix mit einem Flächengewicht von 96,3 g/m² resultiert. Auf die so erhaltene Matrix wird eine für die Wirkstoffe undurchlässige Rückschicht (0,015 mm dicke Polyesterfolie) aufkaschiert. Anschließend werden 40 cm² große transdermale therapeutische Systeme ausgestanzt.

### Beispiele 2, 3 und 4:

Die Herstellung erfolgt, wie unter Beispiel 1 beschrieben, jedoch mit den in Tabelle 1 angegebenen Rohstoffen und Mengen.

**Tabelle 1:**

| Zusammensetzung [g] | | | |
|---|---|---|---|
| Beispiel | 2 | 3 | 4 (Vergleichsbeispiel) |
| Durotak 387-2287 | 155,08 | 126,97 | 424,31 |
| Eudragit E 100® | 4,81 | 19,26 | -- |
| Eutanol G | 2,17 | 2,17 | 5,59 |
| Aluminiumacetylacetonat | 0,03 | 0,03 | 1,36 |
| Estradiol-Hemihydrat | 1,29 | 1,29 | 3,37 |
| Norethisteronacetat | 8,33 | 8,33 | 21,60 |
| Ethylacetat | 27,98 | 34,81 | -- |
| Ethanol | 27,97 | 34,80 | -- |
| Methylethylketon | | | 134,79 |

Die Prüfung auf Rekristallisationserscheinungen wurde mikroskopisch bei 40-facher Vergrößerung im Durchlicht durchgeführt. Die Ergebnisse sind in Tabelle 2 dargestellt.

**Tabelle 2:**

| Rekristallisationserscheinungen | |
|---|---|
| Beispiel 1 | Kristalle pro 40 cm² nach einer Lagerung von 3 Monaten bei 40 °C |
| 1 | 1 |
| 2 | 1 |
| 3 | 0 |
| 4 (Vergleichsbeispiel ohne Eudragit E100) | 154 |

Wie die Tabelle 2 zeigt, erhält man durch den Zusatz des Kristallisationsinhibitors Eudragit
E100 transdermale therapeutische Systeme, die kristallisationsfrei sind, im Gegensatz zu dem Vergleichsbeispiel (ohne Eudragit E 100), bei dem es innerhalb eines Zeitraumes von 3 Monaten zu erheblicher Kristallisation kommt.

## Patentansprüche

1. Transdermales therapeutisches System in Pflasterform zur kontrollierten Abgabe von Estradiol in Kombination mit Norethisteronacetat, umfassend eine Rückschicht, ein damit verbundenes, an Wirkstoffen übersättigtes Reservoir, das die Wirkstoffe Estradiol und Norethisteronacetat enthält und das unter Verwendung von Polyacrylat-Haftklebern und Kristallisationsinhibitoren hergestellt ist, und eine wiederablösbare Schutzschicht, **dadurch gekennzeichnet, daß** der Kristallisationsinhibitor ein Terpolymer auf Basis von Butylmethacrylat, 2-Dimethylaminoethylmethacrylat und Methylmethacrylat, insbesondere im molaren Verhältnis von 1 : 2 : 1 ist.

2. Transdermales therapeutisches System nach Anspruch 1, **dadurch gekennzeichnet, daß** das Reservoir den Kristallisationsinhibitor in einem Anteil von 0,05 - 30 Gaw.% enthält.

3. Transdermales therapeutisches System nach Anspruch 1 oder 2, **dadurch gekennzeichnet, daß** das Reservoir Estradiol und Norethisteronacetat in einem Gewichtsverhältnis von 1 : 2 bis 1 : 15, vorzugsweise 1 : 3 bis 1 : 7, und einer Gesamtkonzentration von bis zu 25 Gew.% enthält.

4. Transdermales therapeutisches System nach einem oder mehreren der Ansprüche 1 - 3, **dadurch gekennzeichnet, daß** das Reservoir einen Bestandteil aus der Gruppe der Alterungsschutzmittel, Weichmacher, Antioxydantien und Absorptionsverbesserer enthält, wobei der Weichmacher in einer Konzentration von 0 - 5 Gew.% und das Alterungsschutzmittel in einer Konzentration von 0,1 - 2 Gem.% eingesetzt werden.

5. Transdermales therapeutisches System nach einem oder mehreren der Ansprüche 1 - 4, **dadurch gekennzeichnet, daß** der Haftkleber ein Lösemittelhaftkleber, ein Dispersionshaftkleber, ein Schmelzhaftkleber oder ein mit UV-Strahlen vernetzbarer Kleber ist.

6. Transdermales therapeutisches System nach einem oder mehreren der Ansprüche 1 - 5, **dadurch gekennzeichnet, daß** das Reservoir aus mehreren Schichten besteht.

7. Transdermales therapeutisches System nach einem oder mehreren der Ansprüche 1 - 6, **dadurch gekennzeichnet, daß** das Reservoir eine Schichtdicke von 0,02 mm - 0,500 mm, vorzugsweise von 0,030 - 0,200 mm, aufweist.

8. Transdermales therapeutisches System nach einem oder mehreren der Ansprüche 1 - 7, **dadurch gekennzeichnet, daß** das Reservoir mit einer zusätzlichen haftklebenden Schichtoder einem haftklebenden Rand versehen ist.

## Claims

1. Transdermal therapeutic system in the form of a patch, for the controlled release of estradiol in combination with norethisterone acetate, comprising a backing layer, a reservoir which is bonded thereto and which is supersaturated with active substances, said reservoir containing the active substances, estradiol and norethisterone acetate, said reservoir being produced using pressure-sensitive polyacrylate adhesives and crystallization inhibitors, and a removable protective layer, **characterized in that** the crystallization inhibitor is a terpolymer on the basis of butyl methacrylate, 2-dimethyl aminoethyl methacrylate and methyl methacrylate, especially with a molar ratio of 1 : 2 : 1.

2. Transdermal therapeutic system according to claim 1, **characterized in that** the reservoir contains the crystallization inhibitor in a proportion of 0.05 to 30 wt-%.

3. Transdermal therapeutic system according to claim 1 or 2, **characterized in that** the reservoir contains estradiol and norethisterone acetate in a weight ratio of 1 : 2 to 1 : 15, preferably 1 : 3 to 1 : 7, and in a total concentration of up to 25 wt-%.

4. Transdermal therapeutic system according to one or more of claims 1 to 3, **characterized in that** the reservoir contains a component of the group of age protecting agents, softeners, antioxidants and absorption enhancers, said softener being used at a concentration of 0 to 5 wt-% and said age protecting agent being used at a concentration of 0.1 to 2 wt-%.

5. Transdermal therapeutic system according to one or more of claims 1 to 4, **characterized in that** the pressure-sensitive adhesive is a pressure-sensitive solvent adhesive, a pressure-sensitive dispersion adhesive, a pressure-sensitive hot-melt adhesive or an adhesive cross-linkable by UV radiation.

6. Transdermal therapeutic system according to one or more of claims 1 to 5, **characterized in that** the reservoir consists of several layers.

7. Transdermal therapeutic system according to one or more of claims 1 to 6, **characterized in that** the reservoir has a layer thickness of 0.02 mm to 0.500 mm, preferably of 0.030 mm to 0.200 mm.

8. Transdermal therapeutic system according to one or more of claims 1 to 7, **characterized in that** the reservoir is provided with an additional pressure-sensitive adhesive layer or with a pressure-sensitive adhesive edge.

## Revendications

1. Système thérapeutique transdermique sous forme de pansement pour la délivrance contrôlée d'oestradiol en association avec de l'acétate de noréthistérone, comprenant une couche dorsale, un réservoir sursaturé en principes actifs, lié à celle-ci, qui contient les principes actifs, l'oestradiol et l'acétate de noréthistérone et qui est fabriqué en employant des colles auto-adhésives à base de polyacrylate et des inhibiteurs de cristallisation, et une couche de protection détachable à répétition, **caractérisé en ce que** l'inhibiteur de cristallisation est un terpolymère à base de méthacrylate de butyle, de méthacrylate de 2-diméthylaminoéthyle et de méthacrylate de méthyle, en particulier dans le rapport molaire de 1:2:1.

2. Système thérapeutique transdermique selon la revendication 1, **caractérisé en ce que** le réservoir contient l'inhibiteur de cristallisation en une proportion de 0,05 à 30% en poids.

3. Système thérapeutique transdermique selon la revendication 1 ou 2, **caractérisé en ce que** le réservoir contient l'oestradiol et l'acétate de noréthistérone dans un rapport pondéral de 1:2 à 1:15, de préférence de 1:3 à 1:7 et à une concentration totale allant jusqu'à 25% en poids.

4. Système thérapeutique transdermique selon l'une ou plusieurs des revendications 1 à 3, **caractérisé en ce que** le réservoir contient un composant du groupe formé par les agents protecteurs contre le vieillissement, les plastifiants, les anti-oxydants et les agents améliorant l'absorption, le plastifiant étant mis en oeuvre en une concentration de 0 à 5% en poids et l'agent protecteur contre le vieillissement, en une concentration de 0,1 à 2% en poids.

5. Système thérapeutique transdermique selon l'une ou plusieurs des revendications 1 à 4, **caractérisé en ce que** la colle auto-adhésive est une colle auto-adhésive à solvant, une colle auto-adhésive à dispersion, une colle auto-adhésive à chaud, ou une colle réticulable par des rayons UV.

6. Système thérapeutique transdermique selon l'une ou plusieurs des revendications 1 à 5, **caractérisé en ce que** le réservoir est constitué de plusieurs couches.

7. Système thérapeutique transdermique selon l'une ou plusieurs des revendications 1 à 6, **caractérisé en ce que** le réservoir présente une épaisseur de couche de 0,02 mm à 0,500 mm, de préférence de 0,030 à 0,200 mm.

8. Système thérapeutique transdermique selon l'une ou plusieurs des revendications 1 à 7, **caractérisé en ce que** le réservoir est muni d'une couche auto-adhésive supplémentaire ou d'un bord auto-adhésif.
